(19) Europäisches Patentamt
European Patent Office
Office européen des brevets

(11) EP 1 459 750 B1

(12) EUROPEAN PATENT SPECIFICATION

(45) Date of publication and mention of the grant of the patent:
**01.06.2005 Bulletin 2005/22**

(51) Int Cl.[7]: **A61K 31/5375**, A61P 29/00, A61P 25/00

(21) Application number: **04013382.9**

(22) Date of filing: **22.06.2000**

(54) **(S,S)-reboxetine for treating fibromyalgia and other somatoform disorders**

(S,S)-Reboxetin zur Behandlung von Fibromyalgie und anderen somatoformen Störungen

(S,S)-réboxétine pour traiter les fibromyalgies et d'autres troubles somatoformes

(84) Designated Contracting States:
**AT BE CH CY DE DK ES FI FR GB GR IE IT LI LU MC NL PT SE**
Designated Extension States:
**AL LT LV MK RO SI**

(30) Priority: **01.07.1999 US 141968 P**
**16.07.1999 US 144131 P**
**06.10.1999 US 158256 P**
**13.12.1999 US 170381 P**

(43) Date of publication of application:
**22.09.2004 Bulletin 2004/39**

(62) Document number(s) of the earlier application(s) in accordance with Art. 76 EPC:
**00941659.5 / 1 196 172**

(73) Proprietor: **Pharmacia & Upjohn Company LLC**
**Kalamazoo, MI 49001 (US)**

(72) Inventors:
- **Ahmed, Saeeduddin**
  **Portage Michigan 49002 (US)**
- **Birgerson, Lars**
  **Martinsville New Jersey 08836 (US)**
- **Cetera, Pasquale**
  **Annandale New Jersey 08801 (US)**
- **Marshall, Robert Clyde**
  **Mattawan Michigan 49071 (US)**
- **McArthur, Robert**
  **Kalamazoo Michigan 49009 (US)**
- **Taylor, Duncan P.**
  **Kalamazoo Michigan 49009 (US)**
- **Wong, Erik H.F.**
  **Portage Michigan 49024 (US)**

(74) Representative: **Duncan, Garreth Andrew**
**Pfizer Limited,**
**UK Patent Department,**
**Ramsgate Road**
**Sandwich, Kent CT13 9NJ (GB)**

(56) References cited:
**WO-A-00/00120** **WO-A-01/47503**
**WO-A-01/62236** **GB-A- 2 167 407**

- **FRIGERIO E ET AL: "PHARMACOKINETICS OF REBOXETINE ENANTIOMERS IN THE DOG" CHIRALITY, WILEY-LISS, NEW YORK, US, vol. 9, 1997, pages 303-306, XP000991008 ISSN: 0899-0042**
- **DOSTERT P ET AL: "REVIEW OF THE PHARMACOKINETICS AND METABOLISM OF REBOXETINE, A SELECTIVE NORADRENALINE REUPTAKE INHIBITOR" EUROPEAN NEUROPSYCHOPHARMACOLOGY, ELSEVIER SCIENCE PUBLISHERS BV, AMSTERDAM, NL, vol. 7, no. SUPPL 1, 1997, pages S23-S35, XP000989913 ISSN: 0924-977X**
- **BURROWS G D ET AL: "ANTIDEPRESSANT EFFICACY AND TOLERABILITY OF THE SELECTIVE NOREPINEPHRINE REUPTAKE INHIBITOR REBOXETINE: A REVIEW" JOURNAL OF CLINICAL PSYCHIATRY, XX, XX, vol. 59, no. SUPPL 14, 1998, pages 4-7, XP000989995 ISSN: 0160-6689**
- **ANONYMOUS: "REBOXETINE - ANOTHER NEW ANTIDEPRESSANT" DRUG AND THERAPEUTICS BULLETIN, CONSUMERS ASSOCIATION, LONDON, GB, vol. 36, no. 11, November 1998 (1998-11), pages 86-88, XP001011361 ISSN: 0012-6543**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### Field of the Invention

The present invention relates to the use of (S,S) reboxetine, or a pharmaceutically acceptable salt thereof, in the preparation of a medicament for the treatment or prevention of Abromyalgia and other somatoform disorders.

### Brief Description of Related Technology

Many types of depression, mental, behavioral, and neurological disorders originate from disturbances in brain circuits that convey signals using certain monoamine neurotransmitters. Monoamine neurotransmitters include, for example, norepinephrine (noradrenaline), serotonin (5-HT), and dopamine. Lower-than-normal levels of norepinephrine are associated with a variety of symptoms including lack of energy, motivation, and interest in life. Thus, a normal level of norepinephrine is essential to maintaining drive and capacity for reward.

These neurotransmitters travel from the terminal of a neuron across a small gap (*i.e.,* the synaptic cleft) and bind to receptor molecules on the surface of a second neuron. This binding elicits intracellular changes that initiate or activate a response or change in the postsynaptic neuron. Inactivation occurs primarily by transport (*i.e.,* reuptake) of the neurotransmitter back into the presynaptic neuron. Abnormality in noradrenergic transmission results in various types of depression, mental, behavioral, and neurological disorders attributed to a variety of symptoms including a lack of energy, motivation, and interest in life. *See generally*, R.J. Baldessarini, "Drugs and the Treatment of Psychiatric Disorders: Depression and Mania" in *Goodman and Gilman's The Pharmacological Basis of Therapeutics,* McGraw-Hill, NY, NY, pp. 432-439 (1996).

Reboxetine (*i.e.*, 2-[(2-ethoxyphenoxy)(phenyl)methyl] morpholine) raises the concentration of physiologically active norepinephrine by preventing reuptake of norepinephrine, for example. Reboxetine is a norepinephrine reuptake inhibitor and has been shown to be effective in the short-term (*i.e.*, less than eight weeks) and long-term treatment of depression. In fact, reboxetine has been shown to have effectiveness that is similar to fluoxetine, imipramine, and desipramine, commonly prescribed antidepressants, in both adults and elderly patients. *See* S.A. Montgomery, *Reboxetine: Additional Benefits to the Depressed Patient,* Psychopharmocol (Oxf) 11:4 Suppl., S9-15 (Abstract) (1997).

Antidepressant drugs are sometimes divided into "generations." The first generation included the monoamine oxidase inhibitors (such as isocarboxazid and phenylhydrazine) and tricyclic agents (such as imipramine). The second generation of antidepressant drugs included compounds such as mianserin and trazodone. The third generation has included drugs called selective reuptake inhibitors (*e.g.*, fluoxetine, sertraline, paroxetine, and reboxetine). Those drugs were characterized by relatively selective action on only one of the three main monoamine systems thought to be involved in depression (*i.e.*, 5-HT (serotonin), noradrenaline (norepinephrine), and dopamine). APP Textbook of Psychopharmacology (A.F. Schatzberg and C.B. Nemeroff), American Psychiatric Press, 2d. ed., (1998); Lexicon of Psychiatry, Nuerology and the Neurosciences (F.J. Ayd, Jr.) Williams and Wilkins (1995). The antidepressant efficacy of reboxetine is evidenced by its ability to prevent resperine-induced blepharospasm and hypothermia in mice, down regulation of β-adrenergic receptors and desensitization of noradrenaline-coupled adenylate cyclase. See M. Brunello and G. Racagni, "Rationale for the Development of Noradrenaline Reuptake Inhibitors," Human Psychophramacology, vol. 13, S-13-519, Supp. 13-519 (1998).

According to a survey by Brian E. Leonard, desipramine, maprotiline, and lofepramine are relatively selective norepinephrine reuptake inhibitors with proven efficacy. These materials increase brain noradrenaline and thereby function to relieve depression. Mianserin and mirtazepine also show antidepressant-like effects by increasing noradrenaline availability by means of blocking the pre-synaptic $\alpha_2$-adrenoceptors. Still further, oxaprotiline, fezolamine, and tomoxetine are potent and selective norepinephrine reuptake inhibitors that lack neurotransmitter receptor interactions and, thus, do not cause many of the side effects characteristic of classical tricyclic antidepressants. *See* Brian E. Leonard, "The Role of Noradrenaline in Depression: A Review," Journal of Psychopharmocology, vol. 11, no. 4 (Suppl.), pp. S39-S47 (1997)

Reboxetine also is a selective norepinephrine reuptake inhibitor, which also produces fewer of the side effects associated with the administration of classical tricyclic antidepressants. The antidepressant efficacy of reboxetine is evidenced by its ability to prevent resperine-induced blepharospasm and hypothermia in mice, down regulation of β-adrenergic receptors and desensitization of noradrenaline-coupled adenylate cyclase. *See* M. Brunello and G. Racagni, "Rationale for the Development of Noradrenaline Reuptake Inhibitors," Human Psychophramacology, vol. 13 (Supp.) 13-519 (1998).

Reboxetine generally is described in Melloni *et al.* U.S. Patent Nos. 4,229,449, 5,068,433, and 5,391,735, and in GB 2,167,407. Chemically, reboxetine has two chiral centers and, therefore, exists as two enantiomeric pairs

of diastereomers, shown below as isomers (I) through (IV):

(I)

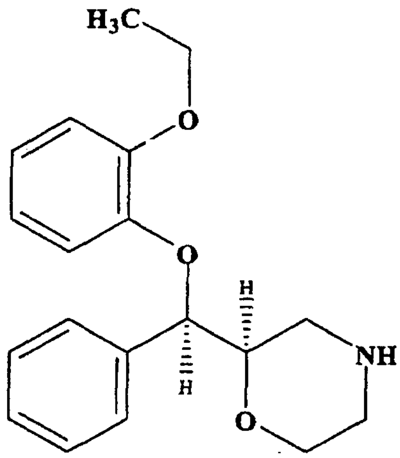

(R, R) 2-[(2-ethoxyphenoxy)(phenyl)methyl]morpholine

(II)

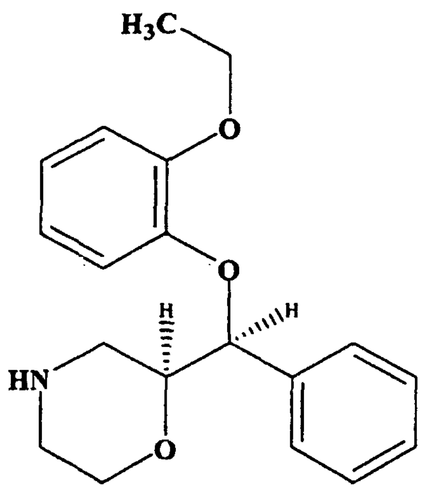

(S, S) 2-[(2-ethoxyphenoxy)(phenyl)methyl]morpholine

(III)

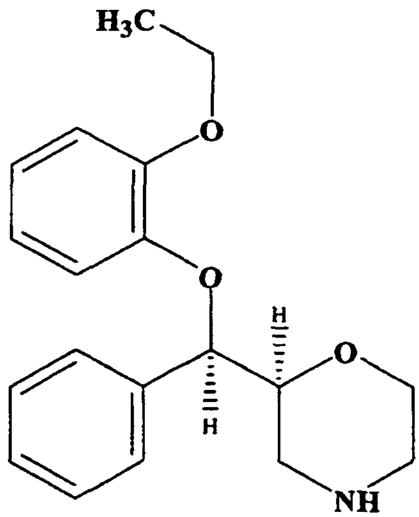

(R, S) 2-[(2-ethoxyphenoxy)(phenyl)methyl]morpholine

(IV)

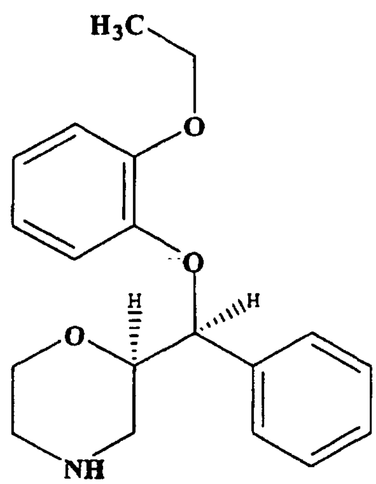

(S, R) 2-[(2-ethoxyphenoxy)(phenyl)methyl]morpholine

Many organic compounds exist in optically active forms, *i.e.*, they have the ability to rotate the plane of plane-polarized light. In describing an optically active compound the prefixes R and S are used to denote the absolute configuration of the molecule about its chiral center(s). The prefixes D and L, or (+) or (-), designate the sign of rotation of plane-polarized light by the compound, with L or (-) meaning that the compound is levorotatory. In contrast, a compound prefixed with D or (+) is dextrorotatory. There is no correlation between nomenclature for the absolute stereochemistry and for the rotation of an enantiomer. Thus, D-lactic acid is the same as (-)-lactic acid, and L-lactic acid is the same as (+)-lactic acid. For a given chemical structure, each of a pair of enantiomers are identical except that they are non-superimposable mirror images of one another. A specific stereoisomer may also be referred to as an enantiomer, and a mixture of such isomers is often called an enantiomeric, or racemic, mixture.

Stereochemical purity is important in the pharmaceutical field, where many of the most often prescribed drugs exhibit chirality. For example, the L-enantiomer of the beta-adrenergic blocking agent, propranolol, is known to be 100 times more potent than its D-enantiomer. Additionally, optical purity is important in the pharmaceutical drug field because certain isomers have been found to impart a deleterious effect, rather than an advantageous or inert effect. For example, it is believed that the D-enantiomer of thalidomide is a safe and effective sedative when prescribed for the control of morning sickness during pregnancy, whereas its corresponding L-enantiomer is believed to be a potent teratogen.

When two chiral centers exist in one molecule, there are four possible stereoisomers: (R,R), (S,S), (R,S), and (S,R). Of these, (R,R) and (S,S) are an example of a pair of enantiomers (mirror images of each other), which typically share chemical properties and melting points just like any other enantiomeric pair. The mirror images of (R,R) and (S, S) are not, however, superimposable on (R,S) and (S,R). This relationship is called diastereoisomeric, and the (S,S) molecule is a diastereoisomer of the (R,S) molecule, whereas the (R,R) molecule is a diastereoisomer of the (S,R) molecule.

Currently, reboxetine is commercially available only as a racemic mixture of enantiomers, (R,R) and (S,S) in a 1:1 ratio, and reference herein to the generic name "reboxetine" refers to this enantiomeric, or racemic, mixture. Reboxetine is commercially sold under the trade names of EDRONAX™, PROLIFT™, VESTRA™, and NOREBOX™. As previously noted, reboxetine has been shown to be useful in the treatment of human depression. Orally administered reboxetine is readily absorbed and requires once or twice a day administration. A preferred adult daily dose is in the range of 8 to 10 milligrams (mg). The effective daily dosage of reboxetine for a child is smaller, typically in a range of 4 to 5 mg. The optimum daily dosage for each patient, however, must be determined by a treating physician taking into account the patient's size, other medications which the patient may be taking, identity and severity of the particular disorder, and all of the other circumstances of the patient.

Administration ofreboxetine, however, can result in undesired side effects associated with drug-drug interactions and in other undesirable effects such as, for example, dizziness, insomnia, lightheadedness, changes in blood pressure, sweating, gastrointestinal disturbances, sexual dysfunction in males, certain anticholinergic-like effects (*e. g.*, tachyardia and urinary retention). It has been found that such side effects occur, in part, because reboxetine lacks a sufficiently high selectivity for inhibiting norepinephrine reuptake. In other words, reboxetine is blocking reuptake of other monoamines, like serotonin and dopamine, to a sufficient degree to contribute to the undesired side effects.

It has been reported that other antidepressants have a high pharmacological selectivity for inhibiting reuptake of norepinephrine. For example, oxaprotiline has a pharmacological selectivity with respect to inhibiting norepinephrine reuptake compared to serotonin reuptake of about 4166, based on a ratio of $K_i$ values. The corresponding pharmacological selectivity for desipramine is about 377, and that for maprotiline is about 446. *See* Elliott Richelson and Michael Pfenning, "Blockade by Antidepressants and Related Compounds of Biogenic Amine Uptake in Rat Brain Synaptosomes: Most Antidepressants Selectively Block Norepinephrine Uptake," European Journal of Pharmacology, vol. 14, pp. 277-286 (1984). Despite the relatively high selectivity of oxaprotiline, desipramine, and maprotiline, these and other known materials undesirably block receptor of other neurotransmitters to a sufficient degree that they also contribute to adverse side effects.

Accordingly, there is a need in the art for the use of (S,S)-reboxetine or a pharmaceutically acceptable salt thereof in the manufacture of a medicament for treating individuals suffering from Abromyalgia and other somatoform disorders where inhibiting reuptake of norepinephrine provides a benefit, while reducing or eliminating the adverse side effects associated with conventional norepinephrine reuptake inhibitors. There also is a need for the use of (S, S)-reboxetine or a pharmaceutically acceptable salt thereof that selectively inhibits the reuptake of norepinephrine over other neurotransmitters, like serotonin and dopamine. Specifically, there is a need in the art for a highly selective (at one reuptake site), specific (with no activity at other receptors), and potent norepinephrine reuptake inhibitor. Furthermore, there is a need for pharmaceutical compositions containing a highly selective and potent norepinephrine reuptake inhibitor. Still further, there is a need for medicaments containing such pharmaceutical compositions, and the use of such compositions in the manufacture of such medicaments.

## SUMMARY OF THE INVENTION

The present invention is directed to the use of optically pure (S,S)-reboxetine, or a pharmaceutically acceptable salt thereof, in the preparation of a medicament to treat or prevent fibromyalgia and other somatoform disorders (including somatization disorder, conversion disorder, pain disorder, hypochondriasis, body dysmorphic disorder, undifferentiated somatoform disorder, and somatoform NOS), wherein the optically pure (S,S)-reboxetine or pharmaceutically acceptable salt thereof comprises at least 90 wt.% of (S,S)-reboxetine and less than 10 wt.% of (R,R)-reboxetine, based on the total weight of the (S,S)-and (R,R)-reboxetine prevent.

Individuals treated with optically pure (S,S) reboxetine do not experience certain adverse side effects associated with the administration of the racemic mixture of (R,R) and (S,S) reboxetine. Administration of optically pure (S,S) reboxetine to a human therefore selectively inhibits norepinephrine reuptake, and thereby controls, reduces, or eliminates adverse effects caused by the administration of the racemic mixture of reboxetine.

(S,S)-reboxetine or a pharmaceutically acceptable salt thereof may be used in a dose of 0.5 to 10 mg/day.

Optically pure (S,S) reboxetine is advantageous over prior treatment or prevention methods which utilized a racemic mixture of (R,R) and (S,S) reboxetine. In particular, it has been found that treatments using compositions containing an optically pure (S,S) reboxetine are about 5 to about 8.5 times more effective at inhibiting the reuptake of norepinephrine than compositions containing the racemic mixture of the (R,R) and (S,S) stereoisomers. Therefore, reuptake blockage can be achieved with much lower dosages. Accordingly, the present invention may permit a substantial reduction in the customary daily dosage of the racemic mixture (*i.e.*, the commercially available reboxetine) by about 50% to about 80% because of the use of an optically pure (S,S) reboxetine. In addition, treatments utilizing the optically pure (S,S) reboxetine may result in fewer undesirable adverse side effects associated with the treatment because of the high selectivity and potency of (S,S) reboxetine with respect to inhibiting the reuptake of norepinephrine.

Additional benefits and features of the present invention will become apparent to those skilled in the art from a review of the following detailed description, taken in conjunction with the example and the appended claims.

## DETAILED DESCRIPTION OF THE PREFERRED EMBODIMENTS

Reboxetine is a known compound that is active on the central nervous system, and has been used as an antidepressant. Heretofore, use of reboxetine has been limited to the treatment of depression, oppositional defiant disorder, attention-deficit/hyperactivity disorder, and conduct disorder. These proposed treatments are disclosed in International Publication Nos. WO 99/15163, WO 95/15176, and WO 99/15177. These treatment methods are limited to administration of a racemic mixture of the (S,S) and (R,R) reboxetine stereoisomers.

Reboxetine does not act like most antidepressants. Unlike tricyclic antidepressants, and even selective serotonin reuptake inhibitors (SSRIs), reboxetine is ineffective in the 8-OH-DPAT hypothermia test, indicating that reboxetine is not a SSRI. Brian E. Leonard, "Noradrenaline in basic models of depression." *European-Neuropsychopharmacol,* 7 Suppl. 1 pp. S11-6 and S71-3 (April 1997). Reboxetine is a selective norepinephrine reuptake inhibitor, with only marginal serotonin and no dopamine reuptake inhibitory activity. Reboxetine displays no anticholinergic binding activity in different animal models, and is substantially devoid of monoamine oxidase (MAO) inhibitory activity. Racemic reboxetine exhibits a pharmacological selectivity of serotonin ($K_i$)/norepinephrine ($K_i$) of about 80. The $K_i$ values are discussed in more detail hereafter.

To determine the degree of selectivity of a compound to bind to the norepinephrine reuptake site, the inhibition constant (or $K_i$ value) of the compound for serotonin reuptake site was divided by the $K_i$ value for norepinephrine reuptake site. A lower value of $K_i$ for norepinephrine reuptake indicates greater binding affinity to norepinephrine receptors. A higher serotonin ($K_i$)/norepinephrine ($K_i$) ratio indicates a greater selectivity for binding the norepinephrine receptor.

Optically pure (S,S)-reboxetine, as defined above, is selective with respect to the norepinephrine reuptake site, but do not cause significant blockade of receptors associated with undesirable side effects *e.g.*, serotonin and dopamine receptors. In other words, a dose of optically pure (S,S)-reboxetine, as defined above, capable of inhibiting the reuptake of norepinephrine, is essentially ineffective in eliciting blockade of other neurotransmitter receptors. Inhibition constants ($K_i$ values), typically reported in units of nanamolars (nM), were calculated from the $IC_{50}$ values according to the method set forth in Y.C. Cheng and W.H. Prusoff, "Relationship Between the Inhibitory Constant ($K_i$) and the Concentration of Inhibitor Which Causes 50% Inhibition ($IC_{50}$) of an Enzymatic Reaction," Biochemical Pharmacology, vol. 22, pp. 3099-3108 (1973).

The present invention is directed to the use of an optically pure (S,S) stereoisomer of reboxetine (as defined above) or a pharmaceutically acceptable salt thereof in the manufacture of a medicament to treat or prevent Abromyalgia and other somatoform disorders. (S,S) Reboxetine is an effective, selective inhibitor of norepinephrine reuptake and, accordingly, dose levels can be substantially reduced in comparison to racemic reboxetine. In addition, individuals treated with an optically pure (S,S) reboxetine do not experience certain adverse effects associated with the administration of the racemic mixture of(R,R) and (S,S) reboxetine.

Yet another embodiment of the present invention is directed to the use of optically pure (S,S)-reboxetine (as defined above) or a pharmaceutically acceptable salt thereof wherein the optically pure (S,S)-reboxetine or pharmaceutically acceptable salt thereof is to be administered in a total dose of 0.1 mg/day to 10 mg/day, more preferably 0.5 to 10 mg/day.

As used herein, the term "reboxetine" refers to the racemic mixture of the (R,R) and (S,S) enantiomers of reboxetine. In contrast, the term "(S,S) reboxetine" refers to only the (S,S) stereoisomer. Similarly, the term "(R,R) reboxetine" refers to only the (R,R) stereoisomer.

The phrase "optically pure (S,S) reboxetine" as used herein, is defined as meaning at least 90 percent by weight (wt.%) of (S,S) reboxetine, and 10 wt.% or less of (R,R) reboxetine. In a preferred embodiment the phrase means that it contains at least 97 wt.% of (S,S) reboxetine, and 3 wt.% or less of (R,R) reboxetine. In a more preferred embodiment, the phrase means that it contains at least 99 wt.% of (S,S) reboxetine, and 1 wt.% or less of (R,R) reboxetine. In a most preferred embodiment, the phrase "optically pure (S,S) reboxetine" as used herein, means that it contains greater than 99 wt.% of (S,S) reboxetine. The foregoing percentages are based upon the total amount of reboxetine present in the composition. The phrases "substantially optically pure (S,S) stereoisomer of reboxetine," "substantially optically pure (S,S) reboxetine," "optically pure (S,S) stereoisomer of reboxetine," and "optically pure (S, S) reboxetine" are also encompassed by the above-described amounts.

The phrases "pharmaceutically acceptable salts" or "a pharmaceutically acceptable salt thereof' refer to salts prepared from pharmaceutically acceptable acids or bases, including organic and inorganic acids and bases. Because the active compound (*i.e.*, (S,S) reboxetine) used in the present invention is basic, salts may be prepared from pharmaceutically acceptable acids. Suitable pharmaceutically acceptable acids include acetic, benzenesulfonic (besylate), benzoic, p-bromophenylsulfonic, camphorsulfonic, carbonic, citric, ethanesulfonic, fumaric, gluconic, glutamic, hydrobromic, hydrochloric, hydroiodic, isethionic, lactic, maleic, malic, mandelic, methanesulfonic (mesylate), mucic, nitric, oxalic, pamoic, pantothenic, phosphoric, succinic, sulfuric, tartaric, p-toluenesulfonic, and the like. Examples of such pharmaceutically acceptable salts of (S,S) reboxetine, thus, include, but are not limited to, acetate, benzoate, β-hydroxybutyrate, bisulfate, bisulfite, bromide, butyne-1,4-dioate, carpoate, chloride, chlorobenzoate, citrate, dihydrogenphosphate, dinitrobenzoate, fumarate, glycollate, heptanoate, hexyne-1,6-dioate, hydroxybenzoate, iodide, lactate, maleate, malonate, mandelate, metaphosphate, methanesulfonate, methoxybenzoate, methylbenzoate, monohydrogenphosphate, naphthalene-1-sulfonate, naphthalene-2-sulfonate, oxalate, phenylbutyrate, phenylproionate, phosphate, phthalate, phylacetate, propanesulfonate, propiolate, propionate, pyrophosphate, pyrosulfate, sebacate, suberate, succinate, sulfate, sulfite, sulfonate, tartrate, xylenesulfonate, and the like. A preferred pharmaceutical salt of (S, S) reboxetine is methanesulfonate (*i.e.*, mesylate), which is prepared using methanesulfonic acid.

The phrases "side effects," "adverse effects," and "adverse side effects" in relation to reboxetine include, dizziness, insomnia, lightheadedness, changes in blood pressure, gastrointestinal disturbances, sexual dysfunction in males, extrapyramidal side effects, certain anticholinergic-like effects (*e.g.*, tachycardia, blurred vision), and undesired side effects associated with with drug-drug interactions.

As used herein, the terms "treat," "treatment," and "treating," refer to: (a) preventing a disease, disorder, or condition from occurring in a human which may be predisposed to the disease, disorder and/or condition but has not yet been diagnosed as having it; (b) inhibiting the disease, disorder, or condition, *i.e.*, arresting its development; and (c) relieving the disease, disorder, or condition, *i.e.*, causing regression of the disease, disorder and/or condition. In other words, the terms "treat," "treatment," and "treating," extend to prophylaxis, in other words "prevent," "prevention," and "preventing," as well as treatment of established conditions. Accordingly, use of the terms "prevent," "prevention," and "preventing," would be an administration of the pharmaceutical composition to a person who has in the past suffered from the aforementioned conditions, but is not suffering from the conditions at the moment of the composition's administration. For the sake of simplicity, the term "conditions" as used hereinafter encompasses conditions, diseases, and disorders.

According to the present invention, (S,S)-reboxetine or a pharmaceutically acceptable salt thereof is useful in treating Abromyalgia and other somatoform disorders wherein inhibiting reuptake of norepinephrine provides a benefit. (S,S)-reboxetine or a phamaceutically acceptable salt thereof may be administered and preferably orally administered, in a sufficient amount to provide a total dose of 0.1 to 10 mg/day of the selective compound to an individual.

Administration of a composition containing an optically pure (S,S) reboxetine as defined above is effective in treating fibromyalgia and other somatoform disorders (including somatization disorder, conversion disorder, pain disorder, hypochondriasis, body dysmorphic disorder, undifferentiated somatoform disorder, and somatoform NOS).

The inventive composition includes (S,S) reboxetine. It is known that commercially-available reboxetine is a racemic mixture of the (R,R) and (S,S) enantiomers of 2-[(2-ethoxyphenoxy)(phenyl)methyl]morpholine. It has now been discovered that the (S,S) stereoisomer is the most active and the most selective stereoisomer with respect to inhibiting the reuptake of norepinephrine. In addition, when administered to an individual, in the dosages described herein, as an optically pure material (*i.e.*, in the substantial absence of its (R,R) diastereomer), the individual does not experience many of the adverse side effects associated with the administration of commercially-available reboxetine. Furthermore, it has further been discovered that the (S,S) and (R,R) enantiomers have a reversed selectivity for the norepinephrine neurotransmitter in relation to the serotonin neurotransmitter, and an optically pure (S,S) reboxetine is significantly more effective at inhibiting reuptake of norepinephrine than either the (R,R) enantiomer or a racemic mixture of the (S,S) and (R,R) enantiomers.

Specifically, it has been found that compositions containing an optically pure (S,S) reboxetine are about 5 to about 8.5 times more effective at inhibiting the reuptake of norepinephrine than compositions containing the racemic mixture of the (R,R) and (S,S) stereoisomers. Accordingly, the typical daily dosage of the racemic mixture (*i.e.*, commercially available reboxetine) can be reduced by about 50% to about 80% when using an optically pure (S,S) reboxetine. The reduction in dosage does not lead to a reduction in efficacy, but the reduction or elimination of various adverse side effects was observed.

In particular, because an optically pure (S,S) reboxetine selectively inhibits norepinephrine reuptake compared to serotonin reuptake, adverse side effects associated with serotonin reuptake are reduced or eliminated. Such adverse side effects include, but are not limited to, gastrointestinal disturbances, anxiety, sexual dysfunction, and undesirable side effects associated with drug-drug interactions.

The synthesis of a racemic mixture of reboxetine is disclosed in Melloni *et al*. U.S. Patent No. 4,229,449. Individual stereoisomers of reboxetine can be obtained by resolution of the racemic mixture of enantiomers using conventional methods generally known by those skilled in the art. Such methods include resolution by simple crystallization and chromatographic techniques, for example, as set forth in GB 2,167,407.

While it is possible to administer (S,S)-reboxetine or a pharmaceutically acceptable salt thereof directly without any formulation, a composition preferably is administered in the form of pharmaceutical medicaments comprising (S,S)-reboxetine or a pharmaceutically acceptable salt thereof. The inventive composition can be administered in oral unit dosage forms such as tablets, capsules, pills, powders, or granules. The inventive composition also can be introduced parenterally, (*e.g.*, subcutaneously, intravenously, or intramuscularly), using forms known in the pharmaceutical art. The inventive composition further can be administered rectally or vaginally in such forms as suppositories or bougies. The inventive composition also can be administered topically or transdermally, such as with a "patch" containing active ingredient. Transdermal delivery patches can be used to provide continuous, pulsatile, or on-demand infusion of the inventive compositions in controlled amounts. The construction and use of transdermal delivery patches are well known in the pharmaceutical art, and are described, for example, in U.S. Patent Nos. 3,742,951, 3,742,951, 3,797,494, 3,996,934,4,031,894, and 5,023,252.

It may be desirable or necessary to introduce the inventive composition or pharmaceutical compositions containing (S,S)-reboxetine or a pharmaceutically acceptable salt thereof to the brain, either directly or indirectly. Direct techniques usually involve placement of a suitable drug delivery catheter into the ventricular system to bypass the blood-brain barrier. One such suitable delivery system used for the transport of biological factors to specific anatomical regions of the body is described in U.S. Patent No. 5,011,472.

In general, the preferred route of administering the inventive composition is oral, with a once or twice a day administration. The dosage regimen and amount for treating patients with the inventive composition is selected in accordance with a variety of factors including, for example, the type, age, weight, sex, and medical condition of the patient, the severity of the condition, the route of administration and the particular compound employed, either racemate or pure enantiomer. An ordinarily skilled physician or psychiatrist can readily determine and prescribe an effective (*i. e*., therapeutic) amount of the compound to prevent or arrest the progress of the condition. In so proceeding, the physician or psychiatrist could employ relatively low dosages at first, subsequently increasing the dose until a maximum response is obtained.

Pharmaceutical compositions suitable for oral administration can be of any convenient form, such as sachets, tablets, capsules, pills, or aerosol sprays, each containing a predetermined amount of the active compound either as a powder or granules, or as a solution or a suspension in an aqueous liquid, a non-aqueous liquid, an oil-in-water emulsion, or a water-in-oil liquid emulsion. Such compositions can be prepared by any method that includes the step of bringing the active compound either into intimate association with a carrier, which constitutes one or more necessary or desirable ingredients. Generally, the compositions are prepared by uniformly and intimately admixing the active ingredient with liquid carriers or finely divided solid carriers or both, and then, if necessary, shaping the product into a desired form.

For example, a tablet can be prepared by compression or molding techniques, optionally, using one or more accessory ingredients. Compressed tablets can be prepared by compressing the active ingredient in a suitable machine into a free-flowing form, such as a powder or granules. Thereafter, the compressed, free-flowing form optionally can be mixed with a binders, diluents, lubricants, disintegrating agents, effervescing agents, dyestuffs, sweeteners, wetting agents, and non-toxic and pharmacologically inactive substances typically present in pharmaceutical compositions. Molded tablets can be made by molding a mixture of the powdered compound moistened with an inert liquid diluent in a suitable machine.

Suitable binders for use in the pharmaceutical preparation include, for example, starches, gelatine, methylcellulose, gum arabic, tragacanth, and polyvinylpyrrolidone. Suitable diluents for use in the pharmaceutical preparation include, for example, lactose, dextrose, sucrose, mannitol, sorbitol, and cellulose. Suitable lubricants for use in the pharmaceutical preparation include, for example, silica, talc, stearic acid, magnesium or calcium stearate, and or polyethylene glycols. Suitable disintegrating agents for use in the pharmaceutical preparation include, for example, starches, alginic acid, and alginates. Suitable wetting agents for use in the pharmaceutical preparation include, for example, lecithin, polysorbates, and laurylsulfates. Generally, any effervescing agents, dyestuffs, and/or sweeteners known by those of ordinary skill in the art can be used in the preparation of a pharmaceutical composition.

Desirably, daily dose of the composition (*e.g.*, tablet, sachet, or capsule) contains from 0.1 to 10 mg of optically pure (S,S) reboxetine, as defined above. More preferably, each dose of the composite contains 0.5 to 8 mg of the active ingredient, optically-pure (S,S) reboxetine, as defined above. Even more preferably, however, each dose contains from 0.5 to 5 mg of the active ingredient, optically-pure (S,S) reboxetine, as defined above. This dosage form permits the full daily dosage of 0.5 to 2.5 mg to be administered in one or two oral doses. This will allow for tablets containing 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9. 2.0, 2.1, 2.2, 2.3, 2.4, or 2.5 mg of optically pure (S,S) reboxetine.

In another embodiment, a preferred daily dose of the composition (*e.g.*, tablet, sachet, or capsule) contains from 0.1 to 0.9 mg of optically pure (S,S) reboxetine, as defined above. More preferably, each dose of the composition contains 0.5 to 0.8 mg of the active ingredient, optically-pure (S,S) reboxetine, as defined above. Even more preferably, however, each dose contains from 0.5 to 0.75 mg of the active ingredient, optically pure (S,S) reboxetine, as defined above. This dosage form permits the full daily dosage of 0.5 to 0.9 mg to be administered in one oral dose.

The use of the present invention is effective in the treatment of child, adolescent, and adult patients. For purposes of the present invention, a child is considered to be a person below the age of puberty, an adolescent is considered to be a person between the age of puberty and up to about 18 years of age, and an adult generally is a person of at least about 18 years of age. As previously noted, the optimum daily dosage for each patient must be determined by a treating physician taking into account each patient's size, other medications which the patient is taking, identity and severity of the disorder, and all of the other circumstances of the patient.

## EXAMPLE

This example demonstrates the superior pharmacological selectivity and potency of a composition according to the present invention. More specifically, this example demonstrates the superior pharmacological selectivity and potency of (S,S) reboxetine compared to its (R,R) stereoisomer and to racemic reboxetine.

Sprague-Dawley rats weighing about 250 to about 300 grams (g) were decapitated, and cerebral cortical tissue was removed immediately. Cerebral cortices were homogenized in nine volumes of medium each containing 0.32 molar (M) sucrose using a rotating pestle. The obtained homogenate was centrifuged at about 1000 x g for about 10 minutes at about 4°C. A supernatant was collected and further centrifuged at about 20,000 x g for about 20 minutes at a temperature of about 4°C. A protein pellet resulting from the centrifuge steps was re-suspended in a Kreb's-Hepes buffer to result in a protein concentration of about 2 mg/ml of buffer. The buffer was maintained at a pH of about 7.0 and contained: 20 mM Hepes; 4.16 mM $NaHCO_3$; 0.44 mM $KH_2PO_4$; 0.63 mM $NaH_2PO_4$; 127 mM NaCl; 5.36 mM KCl; 1.26 mM $CaCl_2$; and 0.98 mM $MgCl_2$.

Protein/buffer suspension was introduced into 166 assay tubes such that about 30 μg ($10^{-6}$ grams) to about 150 μg of the protein was added to each of 166 assay tubes (*i.e.*, 80 assays per transporter assay). Binding to serotonin and norepinephrine reuptake sites was determined as follows. Synaptosomal uptake of $^3$H-norpinephrine was determined as follows. About 1.4 nanomolar of [$^3$H]citalopram and about 1.9 nM of [$^3$H]nisoxetine were used to label serotonin and norepinephrine reuptake sites, respectively. Nonspecific binding was defined by 100 micromolar (μM) fluoxetine (for serotonin) and 10 μM desipramine (for norepinephrine). Incubation in total assay volume of about 500 microliters (μl) was carried out for about 60 minutes (for serotonin) and 120 minutes (for norepinephrine). Both incubations were carried out at about 25°C, and terminated by rapid filtration through a 48-well cell harvester though GFB filters (pre-soaked with about 0.5 PEI for about 4 hours) in a 3 x 5 ml of ice-cold 200 mM tris-HCl, pH 7.0. Punched-out filters were placed into 7 ml minivials and radioactive assayed by liquid scintillation counting.

The ability of reboxetine (*i.e.*, racemic mixture of (R,R) and (S,S) reboxetine), (R,R) reboxetine, and (S,S) reboxetine to bind to norepinephrine and serotonin reuptake sites was evaluated in binding assays using the two radioligands, [$^3$H]citalopram and [$^3$H]nisoxetine. The concentration of the test compound required to inhibit 50% of the specific binding at the two reuptake sites ($IC_{50}$ values) were determined by non-linear least square regression analysis. A conversion of $IC_{50}$ values to $K_i$ values was performed using the Cheng-Prassoff equation presented below:

$$K_i = IC_{50}/(1 + ([L]/[K_d \text{ of } L])),$$

wherein [L] is the radioligand concentration used in nM, and $K_d$ is the binding affinity of L in nM. *See* Y.C. Cheng and W.H. Prusoff, "Relationship Between the Inhibitory Constant ($K_i$) and the Concentration of Inhibitor Which Causes 50% Inhibition ($IC_{50}$) of an Enzymatic Reaction," Biochemical Pharmacology, vol. 22, pp. 3099-3108 (1973).

The $K_i$ values calculated according to the Cheng-Prassoff equation are provided in the table below:

Table

| Compound | Norepinephrine Reuptake ($K_i$ nM) | Serotonin Reuptake ($K_i$ nM) | Selectivity of $K_i$ of Serotonin/Norepinphrine |
|---|---|---|---|
| (S,S) Reboxetine | 0.23 ± 0.06 | 2937 ± 246 | 12,770 |
| (R,R) Reboxetine | 7.0 ± 1.7 | 104 ± 43 | 15 |
| Reboxetine | 1.6 ± 0.6 | 129 ± 13 | 81 |

The data shows that (S,S) reboxetine is about five to about eight fold more potent than the reboxetine racemate with respect to inhibiting the reuptake of norepinephrine. In addition, racemic reboxetine has an 81 fold selectivity favoring norepinephrine reuptake inhibition over serontonin reuptake inhibition. Unexpectedly, the enantiomeric selectivity of the (S,S) and (R,R) reboxetine stereoisomers with respect to inhibiting the reuptake of norepinephrine and serotonin are quite different. The (S,S) enantiomer is very poor with respect to inhibiting reuptake of serotonin (*i.e.*, a high $K_i$) and, therefore, has a surprisingly high selectivity for the norepinephrine reuptake site. In particular, the selectivity of serotonin versus norepinephrine increases from 81 (for the racemate) to 12,770 for an optically pure (S,S) reboxetine. Accordingly, administration of a therapeutic dose of (S,S) reboxetine effectively inhibits nonepinephrine reuptake, but serotonin reuptake essentially is not affected. Likewise, there is a further increase in separation between the action on norepinephrine reuptake sites and at other receptors. As a consequence, adverse side effects associated with the inhibition of serotonin reuptake and blockade at other receptors are not manifested.

Surprisingly, this effect is not observed with (R,R) reboxetine, but are quite to the contrary. (R,R) reboxetine is a weaker inhibitor than (S,S) reboxetine with respect to nonepinphrine reuptake, *i.e.*, affinity ($K_i$) for (R,R) reboxetine is 7 nM whereas the $K_i$ for (S,S) reboxetine is 0.23 nM. In addition, (R,R) reboxetine is much more effective at inhibiting serotonin uptake than (S,S) reboxetine, *i.e.*, $K_i$ for (R,R) reboxetine is 104 nM, whereas the $K_i$ for (S,S) reboxetine is 2937 nM. Accordingly, (R,R) reboxetine has a low selectivity for nonepinephrine reuptake inhibition versus serotonin reuptake inhibition.

The surprisingly high potency of the (S,S) enantiomer over both the racemic reboxetine and (R,R) reboxetine provides a treating physician an ability to prescribe an effective dosage of a norepinephrine reuptake inhibitor, *i.e.*, (S, S) reboxetine, that is about 10% to about 20% of the current daily dosage of reboxetine (racemate) to achieve the same reuptake inhibition at the norepinephrine site. In addition, the surprisingly high inhibition selectivity of an optically pure (S,S) reboxetine essentially limits inhibition to norepinephrine reuptake, thereby reducing adverse side effects associated with inhibition at serotonin reuptake sites and blockade at other receptors.

## Claims

1. The use of optically pure (S,S)-reboxetine, or a pharmaceutically acceptable salt thereof, in the manufacture of a medicament for the treatment or prevention of fibromyalgia or another somatoform disorder, wherein the optically pure (S,S)-reboxetine or pharmaceutically acceptable salt thereof comprises at least 90 wt.% of (S,S)-reboxetine and less than 10 wt.% of (R,R)-reboxetine, based on the total weight of the (S,S) and (R,R) reboxetine present.

2. The use of claim 1, wherein the (S,S)-reboxetine is to be administered in an amount of 0.1 to 10 mg/day.

3. The use of claim 2, wherein the (S,S)-reboxetine is to be administered in an amount of 0.5 to 8 mg/day, for example 0.5 to 5 mg/day, 0.5 to 2.5 mg/day, 0.5 to 0.9 mg/day, 0.5 to 0.8 mg/day, or 0.5 to 0.75 mg/day.

4. The use of any preceding claim, wherein the pharmaceutically acceptable salt of (S,S)-reboxetine is the methanesulfonate.

5. The use of any preceding claim, wherein the optically pure (S,S)-reboxetine or pharmaceutically acceptable salt thereof comprises at least 97 wt.% of (S,S)-reboxetine and less than 3 wt.% of (R,R)-reboxetine, based on the total weight of the (S,S) and (R,R) reboxetine present.

6. The use of claim 5, wherein the optically pure (S,S)-reboxetine or pharmaceutically acceptable salt thereof comprises at least 99 wt.% of (S,S)-reboxetine and less than 1 wt.% of (R,R)-reboxetine, based on the total weight of the (S,S) and (R,R) reboxetine present.

7. The use of any preceding claim, wherein the somatoform disorder comprises fibromyalgia.

8. The use of any preceding claim, wherein the somatoform disorder comprises somatisation disorder, conversion disorder, pain disorder, hypochondriasis, body dysmorphic disorder, undifferentiated somatoform disorder, or somatoform NOS.

## Patentansprüche

1. Verwendung von optisch reinem (S,S)-Reboxetin oder eines pharmazeutisch akzeptablen Salzes davon zur Herstellung eines Medikamentes zur Behandlung oder Prävention von Fibromyalgie oder einer anderen somatoformen Störung, wobei das optisch reine (S,S)-Reboxetin oder das pharmazeutisch akzeptable Salz davon mindestens 90 Gew.-% (S,S)-Reboxetin und weniger als 10 Gew.-% (R,R)-Reboxetin, bezogen auf das Gesamtgewicht des vorhandenen (S,S)- und (R,R)-Reboxetins, umfasst.

2. Verwendung nach Anspruch 1, wobei das (S,S)-Reboxetin in einer Menge von 0.1 bis 10 mg/Tag zu verabreichen ist.

3. Verwendung nach Anspruch 2, wobei das (S,S)-Reboxetin in einer Menge von 0,5 bis 8 mg/Tag, beispielsweise von 0,5 bis 5 mg/Tag, von 0,5 bis 2,5 mg/Tag, von 0,5 bis 0,9 mg/Tag, von 0,5 bis 0,8 mg/Tag oder von 0,5 bis 0,75 mg/Tag zu verabreichen ist.

4. Verwendung nach irgend einem der vorangehenden Ansprüche, wobei das pharmazeutisch akzeptable Salz von (S,S)-Reboxetin das Methansulfonat ist.

5. Verwendung nach irgend einem der vorangehenden Ansprüche, wobei das optisch reine (S,S)-Reboxetin oder das pharmazeutisch akzeptable Salz davon mindestens 97 Gew.-% (S,S)-Reboxetin und weniger als 3 Gew.-% (R,R)-Reboxetin, bezogen auf das Gesamtgewicht des vorhandenen (S,S)- und (R,R)-Reboxetins, umfasst.

6. Verwendung nach Anspruch 5, wobei das optisch reine (S,S)-Reboxetin oder das pharmazeutisch akzeptable Salz davon mindestens 99 Gew.-% (S,S)-Reboxetin und weniger als 1 Gew.-% (R,R)-Reboxetin, bezogen auf das Gesamtgewicht des vorhandenen (S,S)- und (R,R)-Reboxetins, umfasst.

7. Verwendung nach irgend einem der vorangehenden Ansprüche, wobei die somatoforme Störung eine Fibromyalgie umfasst.

8. Verwendung nach irgend einem der vorangehenden Ansprüche, wobei die somatoforme Störung eine Somatisierungsstörung, eine Konversionsstörung, eine Schmerzstörung, eine Hypochondrie, eine dysmorphe Körperstörung, eine undifferenzierte somatoforme Störung oder eine somatoforme NOS umfasst.

## Revendications

1. Utilisation de (S,S)-réboxétine optiquement pure, ou d'un sel pharmaceutiquement acceptable d'un tel composé, dans la fabrication d'un médicament pour le traitement ou la prévention de la fibromyalgie ou d'un autre trouble somatoforme, dans laquelle la (S,S)-réboxétine optiquement pure ou le sel pharmaceutiquement acceptable d'un tel composé renferme au moins 90 % en poids de (S,S)-réboxétine et moins de 10 % en poids de (R,R)-réboxétine, sur la base du poids total de (S,S) et (R,R)-réboxétine présente.

2. Utilisation selon la revendication 1, dans laquelle la (S,S)-réboxétine doit être administrée à raison de 0,1 à 10 mg/jour.

3. Utilisation selon la revendication 2, dans laquelle la (S,S)-réboxétine est à administrer à raison de 0,5 à 8 mg/jour, par exemple 0,5 à 5 mg/jour, 0,5 à 2,5 mg/jour, 0,5 à 0,9 mg/jour, 0,5 à 0,8 mg/jour ou 0,5 à 0,75 mg/jour.

4. Utilisation selon l'une quelconque des revendications précédentes, dans laquelle le sel pharmaceutiquement acceptable de la (S,S)-réboxétine est le méthanesulfonate.

5. Utilisation selon l'une quelconque des revendications précédentes, dans laquelle la (S,S)-réboxétine optiquement pure ou le sel pharmaceutiquement acceptable d'un tel composé renferme au moins 97 % en poids de (S,S)-réboxétine et moins de 3 % en poids de (R,R)-réboxétine, sur la base du poids total de la (S,S) et (R,R)-réboxétine présente.

6. Utilisation selon la revendication 5, dans laquelle la (S,S)-réboxétine optiquement pure ou le sel pharmaceutiquement acceptable d'un tel composé renferme au moins 99 % en poids de (S,S)-réboxétine et moins de 1 % en poids de (R,R)-réboxétine, sur la base du poids total de la (S,S) et (R,R)-réboxétine présente.

7. Utilisation selon l'une quelconque des revendications précédentes, dans laquelle le trouble somatoforme comprend la fibromyalgie.

8. Utilisation selon l'une quelconque des revendications précédentes, dans laquelle le trouble somatoforme comprend un trouble de somatisation, un trouble de conversion, un trouble douloureux, une hypochondrie, un trouble dysmorphique corporel, un trouble somatoforme indifférencié ou un trouble somatoforme sans autre précision.